# EUROPÄISCHE PATENTSCHRIFT

(11) **EP 3 830 292 B1**
(45) Veröffentlichungstag und Bekanntmachung des Hinweises auf die Patenterteilung: **12.03.2025**
(21) Anmeldenummer: 19749609.4
(22) Anmeldetag: 23.07.2019
(51) Int. Cl.: C12Q 1/6851

(54) **VERFAHREN ZUR DURCHFÜHRUNG EINER ECHTZEIT-PCR**
METHOD FOR CARRYING OUT REAL-TIME PCR
PROCÉDÉ DE RÉALISATION D'UNE PCR EN TEMPS RÉEL

(30) Priorität: 03.08.2018 DE 102018213026
(43) Veröffentlichungstag der Anmeldung: 09.06.2021
(73) Patentinhaber: Robert Bosch GmbH, 70442 Stuttgart (DE)
(72) Erfinder: HOFFMANN, Jochen, 71272 Renningen (DE); FRANK, Tino, 6003 Luzern (CH)
(86) Internationale Anmeldenummer: PCT/EP2019/069741
(87) Internationale Veröffentlichungsnummer: WO 2020/025387

(56) Entgegenhaltungen:
- EP-A1- 3 101 144
- EP-A2- 0 959 140
- EP-A2- 1 138 783
- WO-A1-2007/059179
- WO-A1-2015/109330
- WO-A2-2012/069484
- WO-A2-2014/210199
- DE-A1- 102006 027 675
- US-A1- 2017 106 370
- US-B2- 7 629 151
- US-B2- 8 119 352
- JACQUIE T. KEER: "Qantitative Real-time PCR Analysis", ESSTENTIALS OF NUCLEIC ACID ANALYSIS: A ROBUST APPROACH, 25 February 2008 (2008-02-25), Editors: Jacquie T. Keer and Lyndsey Birch, pages 132 - 166, XP002794168, ISBN: 978-0-85404-367-5, Retrieved from the Internet <URL:https://www.gene-quantification.de/keer-qpcr-book-chapter-7.pdf> [retrieved on 20190910], DOI: 10.1039/9781847558213
- LIU WEIHONG ET AL: "Validation of a quantitative method for real time PCR kinetics", BIOCHEMICAL AND BIOPHYSICAL RESEARCH COMMUNICATIONS, ACADEMIC PRESS, SAN DIEGO, CA; US, vol. 294, no. 2, 7 June 2002 (2002-06-07), pages 347 - 353, XP002594647, ISSN: 0006-291X
- LIU W ET AL: "A New Quantitative Method of Real Time Reverse Transcription Polymerase Chain Reaction Assay Based on Simulation of Polymerase Chain Reaction Kinetics", ANALYTICAL BIOCHEMISTRY, ELSEVIER, AMSTERDAM, NL, vol. 302, no. 1, 1 March 2002 (2002-03-01), pages 52 - 59, XP027227085, ISSN: 0003-2697, [retrieved on 20020301]
- TANIA NOLAN, JIM HUGGET AND ELENA SANCHEZ: "Good practice guide for the application of quantitative PCR (qPCR), LGC (2013)", January 2013 (2013-01-01), pages 1 - 100, XP002794169, Retrieved from the Internet <URL:https://www.gene-quantification.de/national-measurement-system-qpcr-guide.pdf> [retrieved on 20190910]
- KUANG JUJIAO ET AL: "An overview of technical considerations when using quantitative real-time PCR analysis of gene expression in human exercise research.", PLOS ONE 2018, E0196438, vol. 13, no. 5, 10 May 2018 (2018-05-10), pages 1 - 27, XP002794170, ISSN: 1932-6203
- RODRIGO A G ET AL: "QUANTITATION OF TARGET MOLECULES FROM POLYMERASE CHAIN REACTION-BASED LIMITING DILUTION ASSAYS", AIDS RESEARCH AND HUMAN RETROVIRUSES, MARY ANN LIEBERT, US, vol. 13, no. 9, 1 January 1997 (1997-01-01), pages 737 - 742, XP001109652, ISSN: 0889-2229
- ANONYMOUS: "qPCR guide", 10 March 2009 (2009-03-10), pages 1 - 67, XP055381781, Retrieved from the Internet <URL:https://secure.eurogentec.com/uploads/qPCR-guide.pdf> [retrieved on 20170614]
- N.A.: "Real-time PCR handbook", LIFETECHNOLOGIES, 1 January 2014 (2014-01-01), pages 1 - 69, XP002794171, Retrieved from the Internet <URL:https://www.thermofisher.com/content/dam/LifeTech/global/Forms/PDF/real-time-pcr-handbook.pdf> [retrieved on 20160524]
- N/A: "Bulletin 5279B: Real-Time PCR Applications Guide", BIO-RAD LABORATORIES, INC., 1 January 2006 (2006-01-01), pages 1 - 100, XP055423607, Retrieved from the Internet <URL:http://www.bio-rad.com/pdf/Bulletin_5279B.pdf> [retrieved on 20171110]

## Beschreibung

Die vorliegende Erfindung betrifft ein Verfahren zur Durchführung einer Echtzeit-PCR, wobei PCR-Zyklen zur Amplifikation von Proben-Nukleinsäuren und von Referenz-Nukleinsäuren durchgeführt werden. Weiterhin offenbart ist ein Computerprogramm, das zur Durchführung des Verfahrens eingerichtet ist.

### Stand der Technik

Die Polymerase-Kettenreaktion (PCR) ist eine sensitive Methode der Bioanalytik zum Nachweis bestimmter Genabschnitte oder allgemein von NukleinsäureSequenzen. Hierbei werden spezifische DNA-Sequenzen durch zyklisches Duplizieren vervielfältigt bzw. amplifiziert. Für die Vervielfältigung ist das Enzym DNA-Polymerase erforderlich. Die Produkte eines Vervielfältigungszyklus dienen dabei als Ausgangsstoffe beziehungsweise als Vorlage *(template)* für den nächsten Vervielfältigungszyklus. Eine bekannte Ausführungsform der PCR ist die sogenannten Echtzeit-PCR, bei welcher der Reaktionsverlauf insbesondere mittels Fluoreszenzsonden verfolgt werden kann. Eine Echtzeit-PCR erlaubt das Quantifizieren der Ausgangsmenge der DNA, welche sich vor der Amplifikation im Reaktionsgemisch befand. Die Quantifizierung erfolgt auf der Basis von Referenzmessungen, welche bei jeder Reaktion parallel in separaten Reaktionsansätzen mitgeführt und gemessen werden.

Polymerase-Kettenreaktionen laufen in mehreren Amplifikationszyklen ab. Zunächst wird die Ausgangs-DNA denaturiert und dabei in ihre Einzelstränge getrennt (*melting*)*.* In diesem Zustand können Primer im nächsten Schritt sich an die Einzelstränge anlagern (*annealing*)*.* Im folgenden Schritt lagert sich die DNA-Polymerase an und synthetisiert den jeweiligen Gegenstrang der DNA in einer Richtung, beginnend bei den angelagerten Primern (*elongation*)*.* Nach diesem ersten Amplifikationszyklus erfolgen eine erneute Denaturierung und Anlagerung der Primer, gefolgt von einer weiteren Synthese von Gegensträngen. In dem Reaktionsansatz müssen daher DNA-Moleküle als Vorlage, Primer, Nukleotide und das Enzym DNA-Polymerase vorhanden sein. Die Steuerung der Denaturierung, der Primer-Hybridisierung und der Elongation erfolgt über die Einstellung der Temperatur. Der PCR-Prozess wird daher in der Regel in einem Thermocycler durchgeführt, wobei in der Regel etwa 20 bis 50 Amplifikationszyklen vorgesehen sind und die jeweilige Anzahl der Amplifikationszyklen im Vorhinein eingestellt wird.

Die deutsche Offenlegungsschrift DE 10 2010 052 524 A1 beschreibt beispielsweise ein PCR-Verfahren zum qualitativen und quantitativen Nachweis von Nukleinsäuresequenzen in Echtzeit, wobei eine mit einem Fluorophor markierte DNA-Sonde genutzt wird. Mittels Primern wird unter Hybridisierungsbedingungen ein Gemisch von Duplexen erzeugt, an die der markierte Primer angelagert ist. Durch Zugabe einer Polymerase mit Exonukleaseaktivität wird die markierte DNA-Sonde geschnitten und ein Quenching aufgehoben, wodurch ein messbares Fluoreszenz-Signal erzeugt wird.

Die US 8,119,352 B2 beschreibt ein multiplex-Amplifikationsverfahren für Nukleinsäuren. In diesem wir die Amplifikation eines ersten Targetmoleküls analysiert, diese Amplifikation terminiert, sobald ein Schwellenwert erreicht wird, und eine zweite, neue Amplifikation bei einer anderen Schmelztemperatur initiiert.

In der US 2017/0106370 A1 offenbart ein weiteres Amplifikationsverfahren für Nukleinsäuren. In diesem wir die Amplifikation eines ersten Targetmoleküls analysiert und diese Amplifikation bei Erreichen eines vorgegebenen Amplifikationsfortschritts terminiert.

Die EP 3 101 144 A1 beschreibt eine Analysevorrichtung zur Amplifikation und Sequenzierung eines Polynukleotids.

Aus der US 7,629,151 B2 ist ein automatisierter SELEX-Prozess bekannt. In diesem werden alle PCR-Reaktionen in Echtzeit überwacht. Jede PCR-Reaktion kann individuell durch Abkühlung gestoppt werden.

### Offenbarung der Erfindung

### Vorteile der Erfindung

Die vorliegende Offenbarung stellt ein Verfahren zur Durchführung eines Prozesses zur Amplifikation von Nukleinsäuren bereit, wobei Proben-Nukleinsäuren und Referenz-Nukleinsäuren in getrennten Reaktionsansätzen amplifiziert werden. Erfindungsgemäß werden Signale der Amplifikation in Echtzeit beobachtet und die Anzahl von Amplifikationszyklen und/oder die Dauer des Amplifikationsprozesses werden in Abhängigkeit von Signalen der Amplifikation dynamisch eingestellt. Für die Beobachtung der Signale der Amplifikation können die Signale in an sich bekannter Weise detektiert werden, wobei vorzugsweise Fluoreszenzsonden eingesetzt werden, um die erfolgte Amplifikation der Nukleinsäuren detektierbar zu machen. Das System kann dabei so eingerichtet sein, dass die Fluoreszenz proportional mit der Menge der amplifizierten Produkte zunimmt, wobei verschiedene Fluoreszenz-Farbstoffe verwendet werden können. Es können beispielsweise DNA-Farbstoffe wie Cyanin-Farbstoffe (z.B. SYBR^{®} Green oder PicoGreen^{®}) oder Ähnliches verwendet werden, die sich in doppelsträngige DNA einlagern. Eine andere Möglichkeit sind sogenannte FRET-Sonden (Förster-Resonanzenergietransfer), wobei ein Donor-Fluorochrom mit einem Akzeptor-Fluorochrom wechselwirkt. Die detektierten und ausgewerteten Signale der Amplifikation werden in Relation zu den Kontrollen gesetzt und auf dieser Basis werden die Anzahl von Amplifikationszyklen und/oder die Dauer des Amplifikationsprozesses in Abhängigkeit von Signalen der Amplifikation dynamisch eingestellt. Der Kernpunkt der Erfindung liegt also darin, dass die Amplifikationssignale von Probe und Referenz beziehungsweise Kontrolle in Echtzeit beziehungsweise zu mehreren Zeitpunkten während des Ablaufs des Prozesses detektiert und ausgewertet werden und darauf basierend vordefinierte Aktionen ausgeführt werden, indem vor allem die Anzahl der Amplifikationszyklen und/oder die Dauer des Amplifikationsprozesses dynamisch eingestellt werden.

Vorzugsweise handelt es sich bei diesem Prozess um eine Echtzeit-PCR, wobei PCR-Zyklen zur Amplifikation von Proben-Nukleinsäuren und von Referenz-Nukleinsäuren durchgeführt werden. Die Zyklen, deren Anzahl dynamisch eingestellt wird, sind in dieser bevorzugten Ausführungsform PCR-Zyklen. Vorzugsweise werden die Signale der Amplifikation in Bezug zu einem jeweils durchgeführten PCR-Zyklus gesetzt. Es kann also beispielsweise nach jedem PCR-Zyklus eine Detektion und Auswertung der Signale erfolgen. So kann auf der Basis dieser gewissermaßen PCR-simultanen Auswertung beispielsweise nach jedem Zyklus entschieden werden, ob ein erneuter Zyklus gestartet oder der gesamte PCR-Prozess gestoppt werden soll. Wenn beispielsweise ein Signalanstieg bei der Probe mit der Proben-Nukleinsäure und/oder bei dem Ansatz mit der Referenz-Nukleinsäure festgestellt wird, kann die PCR gestoppt werden. Daher kann die Zeit für den PCR-Prozess verkürzt werden, indem der Prozess nach Detektion des Zyklenschwellenwertes (C_{T}-Wert), der den Beginn des exponentiellen Anstiegs des Amplifikationssignals repräsentiert, beendet werden kann. Außerdem kann so die PCR an einem Punkt gestoppt werden, an dem eine definierte und bekannte Menge an PCR-Produkt generiert wurde. Somit kann erreicht werden, dass trotz schwankender PCR-Bedingungen, beispielsweise durch unterschiedliche Beschaffenheit der DNA-enthaltenden Probe, die immer gleiche Produktmenge bei der Amplifikation erzeugt wird.

Darüber hinaus eignet sich das erfindungsgemäße Verfahren auch für andere Amplifikationsprozesse unter Verwendung von DNA-synthetisierenden Enzymen (Amplifikationsenzymen), beispielweise für eine Gesamtgenomamplifikation *(whole genome amplification -* WGA) oder andere, insbesondere auch isotherme, DNA-Amplifikationsmethoden, bei denen der Amplifikationsprozess im Wesentlichen bei gleichbleibender Temperatur abläuft. Bei diesen Prozessen können beispielsweise verschiedene Polymerasen, Helikasen, Ligasen oder Kombinationen von Enzymen des DNA-Replikationsensembles zum Einsatz kommen. In diesen Ausführungsformen wird insbesondere die Dauer des Amplifikationsprozesses in Abhängigkeit von den Amplifikationssignalen dynamisch eingestellt.

Die Beobachtung der Signale der Amplifikation in Echtzeit ist so zu verstehen, dass nicht zwingend durchgängig die Signale detektiert werden, sondern dass zu bestimmten, zeitdiskreten Zeitpunkten, die beispielsweise einzelnen PCR-Zyklen zuzuordnen sind, die Signale detektiert werden können, beispielsweise nach jedem Anlagerungsschritt oder jedem Elongationsschritt eines PCR-Zyklus.

Unter Nukleinsäure ist in diesem Zusammenhang insbesondere DNA zu verstehen, wobei die DNA als Vorlage (*template*) für die Amplifikation dient. Sowohl die Proben-Nukleinsäuren als auch die Referenz-Nukleinsäuren beziehungsweise die Vergleichsproben werden in separaten Reaktionsansätzen mitgeführt. Hierbei enthalten die Reaktionsansätze die jeweilige Nukleinsäure als Template-DNA. Weiterhin sind die üblichen Reagenzien für beispielsweise einen PCR-Ansatz enthalten, also insbesondere Primer, die mit den Einzelsträngen der DNA an bestimmten Positionen infolge der komplementären Nukleotidsequenzen wechselwirken und den Startpunkt der DNA-Synthese festlegen. Weiterhin ist eine thermostabile DNA-Polymerase sowie Desoxyribonukleosid-Triphosphate als Bausteine für den von der DNA-Polymerase zu synthetisierenden DNA-Strang enthalten. Darüber hinaus sind die für die Funktion der DNA-Polymerase erforderlichen Ionen und eine geeignete Pufferlösung vorhanden. Für andere, insbesondere isotherme Amplifikationsprozesse, für die das Verfahren ebenfalls mit Vorteil einsetzbar ist, sind entsprechende, ebenfalls an sich bekannte Komponenten in den Reaktionsansätzen enthalten.

Bei dem Verfahren kann es vorgesehen sein, dass die Beobachtung und/oder die Auswertung der Signale der Amplifikation in Echtzeit erst startet, wenn eine vorgebbare Mindestanzahl von Amplifikationszyklen und/oder eine vorgebbare Mindestdauer des Amplifikationsprozesses durchgeführt worden ist. Beispielsweise diese minimale Zyklenanzahl kann so definiert werden, dass die Zyklenanzahl so gewählt wird, dass vor Ablauf dieser Zyklenzahl bzw. der Mindestdauer der Amplifikation kein Signal zu erwarten ist. Diese Ausgestaltung hat den Vorteil, dass Kapazitäten für die Beobachtung und Auswertung der Signale für die Phasen, in denen keine relevanten Ergebnisse zu erwarten sind, eingespart werden können. Die Mindestanzahl von PCR-Zyklen kann beispielsweise im Bereich von 10 oder weniger liegen. Während dieser Anfangszyklen kann für die nachfolgende Auswertung beispielsweise eine Grundbasislinie generiert werden.

In einer bevorzugten Ausgestaltung des Verfahrens wird der Prozess beendet, wenn die Signalstärke der Amplifikation im Ansatz mit der Proben-Nukleinsäure die Signalstärke der Amplifikation in dem Ansatz mit der Referenz-Nukleinsäure erreicht und/oder übersteigt. In diesem Fall ist davon auszugehen, dass die Menge der Proben-Nukleinsäure der Menge der Referenz-Nukleinsäuren beziehungsweise deren Konzentration entspricht. Mit dieser Ausgestaltung des Verfahrens kann vor allem die Ausgangskonzentration der Proben-Nukleinsäure ermittelt werden und anschließend kann der Prozess beendet werden. Die Beendigung des Prozesses vor dem Erreichen einer vorgebbaren Maximalanzahl von Amplifikationszyklen oder vor einer vorgebbaren Maximaldauer des Prozesses hat den besonderen Vorteil, dass das Auftreten von unerwünschten Nebenprodukten, die vor allem bei hohen Zyklenzahlen am Ende von PCR-Reaktionen entstehen können (z. B. die Bildung von Primer-Dimeren), minimiert wird. Dies erleichtert eine weitergehende Analyse bei der gegebenenfalls vorgesehenen weiteren Charakterisierung der Amplifikationsprodukte.

Der Amplifikationsprozess kann abgebrochen werden, wenn gegebenenfalls eine Maximalanzahl von Amplifikationszyklen und/oder eine vorgebbare Maximaldauer des Prozesses durchgeführt worden ist, ohne dass bis zu diesem Zeitpunkt ein signifikanter Anstieg des Signals der Amplifikation in dem Ansatz mit der Proben-Nukleinsäure festgestellt wurde. Diese Maximalanzahl kann beispielsweise die Anzahl von PCR-Zyklen sein, die in herkömmlichen PCR-Experimenten gewählt wird, beispielsweise 50 PCR-Zyklen.

Insgesamt ist für das vorliegend beschriebene Verfahren keine neue Assay-Entwicklung erforderlich, da die üblichen Reagenzien und Reaktionsparameter für Amplifikationsprozesse verwendet werden. Nur die Steuerung des Prozesses, insbesondere der dynamische Eingriff in die Prozessdauer und beispielsweise in die Anzahl der PCR-Zyklen und gegebenenfalls die Zusammensetzung der Kontrollen, je nach Anwendungsfall, werden in einen neuen Systemzusammenhang gestellt. Dabei erlaubt das beschriebene Verfahren eine kontrollierte volle Automatisierung von Assay-Workflows, ohne dass Quantifizierungsmethoden zwischengeschaltet werden müssten, die eine Probenentnahme mit einer nachfolgenden Aufreinigung der Amplifikationsprodukte benötigen würden.

Das Verfahren kann beispielsweise derart ausgeführt werden, dass die Signale der Amplifikation in Bezug zu jeweils durchgeführten Amplifikationszyklen beobachtet werden. Bei einem signifikanten Anstieg der Signale wird der jeweilige Zyklus als "Amplifikation" eingestuft. Ein Vergleich dieses Einstufungsergebnisses zwischen den Ansätzen mit Proben-Nukleinsäuren und mit Referenz-Nukleinsäuren für den jeweiligen Zyklus wird für eine Auswertung herangezogen. Alternativ (oder zusätzlich) zu einzelnen Amplifikationszyklen können die Signale in Bezug zu festlegbaren Zeitpunkten während des Prozesses gesetzt werden, wobei zu diesen festlegbaren Zeitpunkten die Signale erfasst werden. Beispielsweise können die Signale mit einer Rate zwischen 1 s bis 1 min aufgenommen werden, d.h. dass zum Beispiel mit einer Taktung von 1 s oder 30 s oder 1 min die Signale erfasst (zum Beispiel durch Aufnahme von Fluoreszenzbildern) und beispielsweise wie oben beschrieben ausgewertet werden. Je nach Anwendung kann das Beobachtungszeitfenster beispielsweise zwischen 1 s und 10 min liegen, vorzugsweise zwischen 30 s und 5 min. In einer besonders bevorzugten Ausgestaltung des Verfahrens werden die Ergebnisse des Amplifikationsprozesses als Indikatorenvektordarstellung ausgewertet. Hierfür können als "Amplifikation" eingestuften Amplifikationszyklen oder Zeitpunkte beispielsweise dem Indikatorenwert "1" und die anderen Zyklen oder Zeitpunkte dem Indikatorenwert "0" zugeordnet werden.

Mit dem Verfahren kann in besonders vorteilhafter Weise die Ausgangsmenge der Proben-Nukleinsäuren ermittelt und/oder kontrolliert werden. Hierfür werden parallel vorzugsweise wenigstens zwei Vergleichsproben mit definierter, d.h. bekannter und vorgegebener Ausgangsmenge der Referenz-Nukleinsäuren mitgeführt. Es kann beispielsweise eine Vergleichsprobe mit minimaler Ausgangsmenge beziehungsweise minimaler Ausgangskonzentration und wenigstens eine Vergleichsprobe mit maximaler Ausgangsmenge beziehungsweise maximaler Ausgangskonzentration eingesetzt werden. Die größte Ausgangsmenge (größte Standardkonzentration) und die kleinste beziehungsweise minimale Ausgangsmenge (kleinste Standardkonzentration) erlauben dabei das Einstellen eines Nachweisfensters. Durch weitere Vergleichsproben mit Konzentrationen innerhalb dieses Fensters können mehrere Teilintervalle geschaffen werden, die eine Intervallzuweisung für die Ausgangskonzentration in der Probe erlauben und beispielsweise zur Qualitätskontrolle genutzt werden können. Die verschiedenen Konzentrationen der Vergleichsproben beziehungsweise Standardproben können sich beispielsweise um den Faktor 10 unterscheiden. Sobald in der Probe Amplifikationssignale feststellbar sind (Indikatorenwert "1") kann der Amplifikationsprozess abgebrochen und im Vergleich mit den jeweiligen bis dahin erreichten Indikatorwerten der Ansätze mit den Standardkonzentrationen auf die Ausgangskonzentration bzw. ein Konzentrationsintervall bei der Probe rückgeschlossen werden. Ein besonderer Vorteil hierbei ist, dass die Zeit für die Durchführung des Prozesses verkürzt werden kann. Es muss nicht die maximale Zyklenanzahl oder die maximale Prozessdauer, die bei herkömmlichen Methoden abzuarbeiten ist, durchgeführt werden, um eine Amplifikation und deren Quantität nachweisen zu können, sondern der Prozess kann nach Detektion des Zyklenschwellenwertes (C_{T}-Wert), der den Beginn der exponentiellen Anstiegs des Amplifikationssignals repräsentiert, abgebrochen werden. Die damit verbundene Zeitersparnis ist insbesondere beim Einsatz bei einer *Point-of-Care* (PoC)-Anwendung besonders vorteilhaft.

In einer weiteren bevorzugten Ausgestaltung des Verfahrens wird das Verfahren als Infektionsnachweis eingesetzt. Hierbei wird wenigstens eine Vergleichsprobe mit einer Konzentration der nachzuweisenden Nukleinsäure (beispielsweise ein charakteristischer Genabschnitt eines Pathogens) mitgeführt, die eine untere Nachweisgrenze repräsentiert. Diese Nachweisgrenze kann das späteste Abbruchkriterium der Amplifikationsreaktion darstellen. Wenn in dem Ansatz mit den Proben-Nukleinsäuren früher ein Signal detektiert wird, also insbesondere das Signal "Amplifikation", kann der Test als positiv bewertet werden. Es können hierbei noch weitere Vergleichsproben mit verschiedenen Konzentrationen der nachzuweisenden Nukleinsäure mitgeführt werden, wobei bei einem validen Test die zeitliche Reihenfolge des Auftretens von Amplifikations-Signalen bei den Vergleichsproben der Reihung der Konzentrationen entsprechen sollte.

In einer weiteren Ausgestaltung des Verfahrens wird das Verfahren als Mutationsnachweis eingesetzt. Hierfür werden vorzugsweise eine Vergleichsprobe mit definierter Konzentration der entsprechenden Nukleinsäure, die einen Anteil der nachzuweisenden Mutation von 100% aufweist, sowie vorzugsweise eine weitere Vergleichsprobe mit definierter Konzentration der Nukleinsäure, die einen Anteil der nachzuweisenden Mutation von 0% enthält (Wildtyp), mitgeführt. Zwischen diesen beiden Grenzen können mehrere Mischungsverhältnisse von Mutations-Nukleinsäure und Wildtyp-Nukleinsäure gewählt und eingesetzt werden.

Gemäss vorliegender Erfindung wird das Verfahren wird für eine Gesamtgenomamplifikation (WGA) eingesetzt. Ein besonderer Vorteil hierbei ist, dass die entstandene Produktmenge der Amplifikation kontrolliert werden kann, indem entsprechende Vergleichsreaktionen mit Nukleinsäurekonzentrationen bekannter Konzentration mitgeführt werden. Insbesondere bei Gesamtgenomamplifikationen kann das Problem von unerwünschten Nebenprodukten insbesondere bei hohen Zyklenanzahlen bzw. nach längerer Amplifikationsdauer, also am Ende des WGA-Prozesses, entstehen. Demgegenüber bietet das vorliegend beschriebene Verfahren den Vorteil, dass der Prozess abgebrochen werden kann, sobald eine bestimmte Produktmenge beziehungsweise Produktkonzentration erreicht ist, so dass es nicht zur Bildung von unerwünschten Nebenprodukten kommt beziehungsweise die Bildung von unerwünschten Nebenprodukten minimiert wird.

Für den erfindungsgemässen Einsatz des Verfahrens für eine Gesamtgenomamplifikation wird wenigstens eine Vergleichsprobe mitgeführt, die eine definierte Konzentration der Nukleinsäure (DNA) eines Referenzgenoms enthält. Diese erste Vergleichsprobe ist vorzugsweise für die jeweilige Spezies spezifisch. Wenn z. B. ein menschliches Genom amplifiziert werden soll, wird als Referenzgenom die DNA eines anderen Menschen oder vorzugsweise ein Gemisch aus einer Vielzahl unterschiedlicher Menschen verwendet, sodass der genetischen Vielfalt Rechnung getragen werden kann. Die definierte Konzentration beziehungsweise Menge des Referenzgenoms entspricht, gemäss Erfindung, einer maximal einsetzbaren Menge von DNA für Gesamtgenomamplifikationssysteme. Weiterhin ist vorzugsweise eine zweite Vergleichsprobe vorgesehen, die keine zu amplifizierende Nukleinsäure enthält (no *template control*). Weiterhin ist vorzugsweise eine sogenannte Mengenreferenz als dritte Vergleichsprobe vorgesehen, die eine definierte Menge an Nukleinsäure des Referenzgenoms enthält, wobei diese definierte Menge der gewünschten Zielmenge an Produkt bei der Gesamtgenomamplifikation entspricht. Hierbei enthält dieser Ansatz der dritten Vergleichsprobe kein Amplifikationsenzym. Das heißt, bei dieser dritten Vergleichsprobe findet keine Amplifikation während des Prozesses statt.

Durch Verwendung von Fluoreszenzfarbstoffen, die sich in doppelsträngiger DNA einlagern, die also unabhängig von einer stattfindenden Amplifikation sind, wird bei dieser dritten Vergleichsprobe der Fluoreszenzfarbstoff in die bereits vorliegende doppelsträngige DNA eingelagert, so dass das hieraus resultierende Fluoreszenzsignal dem Signal entspricht, das durch den Prozess bei der eigentlichen Probe für die Gesamtgenomamplifikation erreicht werden soll. Das Auftreten von Amplifikationssignalen bei den Vergleichsproben im Vergleich mit Signalen bei der Probe definiert verschiedene Checkpoints, die eine gesteuerte und automatisierbare Durchführung des Prozesses erlauben.

Das beschriebene Verfahren eignet sich in besonderer Weise für eine Durchführung in mikrofluidischen Systemen, beispielsweise als Lab-on-Chip-System, wobei vorteilhafterweise nur sehr geringe Probenvolumina benötigt werden. Die Vorteile des beschriebenen Systems kommen dabei insbesondere auch im Zusammenhang mit möglichen Automatisierungen zum Tragen.

Die verschiedenen Komponenten für die Durchführung des beschriebenen Verfahrens können beispielsweise als Kit für einen Anwender bereitgestellt werden. Dieser Kit kann dabei insbesondere die für den jeweiligen Prozess erforderlichen Vergleichsproben, Reagenzien, Enzyme und Puffer enthalten.

Das Verfahren kann als Computerprogramm, das zur Durchführung des Verfahrens eingerichtet ist, realisiert sein. Dieses Computerprogramm kann auf einem maschinenlesbaren Datenträger gespeichert sein und/oder in einem entsprechenden Steuergerät für die Durchführung von Amplifikationsprozessen implementiert sein.

Weitere Merkmale und Vorteile der Erfindung ergeben sich aus der nachfolgenden Beschreibung von Ausführungsbeispielen in Verbindung mit den Zeichnungen. Hierbei können die einzelnen Merkmale jeweils für sich oder in Kombination miteinander realisiert sein.

In den Zeichnungen zeigen:
- Fig. 1: schematische Darstellung der Schritte einer Echtzeit-PCR;
- Fig. 2: Illustrierung der Auswertung von Fluoreszenzsignalen bei einem PCR-Prozess;
- Fig. 3: Illustrierung der Durchführung eines Amplifikationsprozesses zur Bestimmung der Anfangskonzentration einer Probe;
- Fig. 4: Auswertung eines Amplifikationsprozesses bei Durchführung eines Infektionsnachweises anhand einer Indikatorenvektordarstellung;
- Fig. 5: Auswertung eines Amplifikationsprozesses bei Durchführung eines Mutationsnachweises anhand einer Indikatorenvektordarstellung;
- Fig. 6: Illustrierung des Ablaufs des erfindungsgemäßen Verfahrens bei einer Gesamtgenomamplifikation und
- Fig. 7: schematische Übersicht über die erforderlichen Gerätekomponenten für die Durchführung einer Echtzeit-PCR gemäß dem erfindungsgemäßen Verfahren.

### Beschreibung von Ausführungsbeispielen

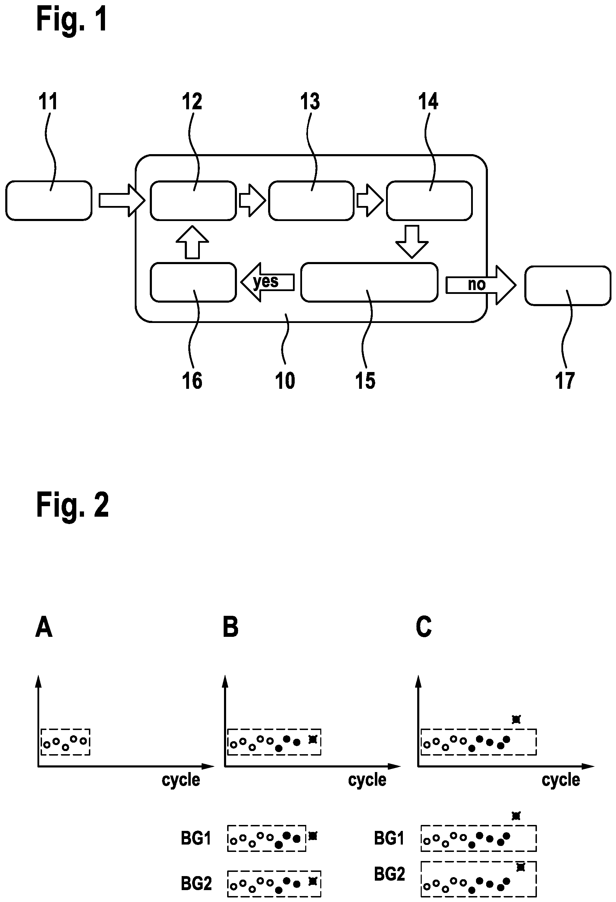

**Fig. 1** zeigt in schematischer Weise den Ablauf einer Echtzeit-PCR 10 gemäß einem Verfahren. Nach dem Start 11 des PCR-Prozesses werden die PCR-Zyklen gestartet, wobei diese einzelnen Schritte durch eine Steuerung der Temperatur in einem Thermocycler vorgenommen werden. In regelmäßigen Intervallen, insbesondere zu definierten Zeitpunkten innerhalb des PCR-Zyklus (bzw. analog zu bestimmten Zeitpunkten bei isothermen Amplifikationsprozessen), werden Signale der Amplifikation erfasst und beispielsweise als Fluoreszenzbilder aufgenommen und ausgewertet. Die Wahl des jeweiligen geeigneten Zeitpunkts kann beispielsweise abhängig von der jeweils verwendeten Sonde sein. In dem hier gezeigten Beispiel wird beispielsweise nach jedem Anlagerungsschritt gemessen. In den meisten Fällen wird jedoch nach jedem Elongationsschritt gemessen. Jeder PCR-Zyklus umfasst den Schritt der Denaturierung 12 der Template-DNA. Als Template-DNA werden in separaten Reaktionsansätzen Proben-Nukleinsäuren und Referenz-Nukleinsäuren verwendet. Nach dem Denaturierungsschritt 12 erfolgt die Anlagerung (*annealing*) der jeweiligen Primer im Schritt 13. Anschließend erfolgt in diesem Beispiel die Messung der Signale der Amplifikation im Schritt 14. Die gemessenen Signale werden im Schritt 15 ausgewertet, wobei insbesondere überprüft wird, ob das gemessene Signal als "Amplifikation" eingestuft wird oder nicht. Insbesondere im Vergleich mit den Referenzproben wird dann entschieden, ob weitere PCR-Zyklen durchgeführt werden oder nicht. Wenn im Schritt 15 beispielsweise festgestellt wird, dass das gemessene Signal als Hintergrund einzustufen ist, also nicht als "Amplifikation", wird der PCR-Zyklus mit dem Elongationsschritt 16 fortgeführt. Anschließend startet wieder der neue PCR-Zyklus mit dem Denaturierungsschritt 12. Wenn im Schritt 15 jedoch festgestellt wird, dass das gemessene Signal nicht als Hintergrundsignal zu werten ist, sondern als "Amplifikation" einzustufen ist, kann der PCR-Prozess abgebrochen werden, wobei gegebenenfalls weitere Analysen und Auswertungen der gebildeten PCR-Produkte erfolgen können (Schritt 17).

Die Detektion der Signale im Schritt 14 basiert auf Fluoreszenzsonden, die auf verschiedene, an sich bekannte Weise, z. B. durch Einbau bei der DNA-Synthese oder durch Anlagerung oder Einlagerung in die DNA eine erfolgte Amplifikation nachweisbar machen. Es wird dann insbesondere statistisch getestet, ob dieser neue Datenpunkt mit schon in vorherigen PCR-Zyklen gemessenen Datenpunkten als Hintergrund klassifiziert werden kann oder ob das Signal signifikant vom bisher bestimmten Hintergrund abweicht und als "Amplifikation" bezeichnet werden kann.

Zweckmäßigerweise werden für den PCR-Prozess eine minimale und eine maximale PCR-Zyklenzahl als Randbedingungen vorgegeben. Mit der minimalen Zyklenanzahl wird definiert, ab wann frühestens ein Signal zu erwarten ist. Diese Datenpunkte werden automatisch dem Hintergrund zugeordnet und werden nicht auf Amplifikation getestet. Diese minimale Zyklenanzahl kann beispielsweise auf 10 oder kleiner festgelegt werden. Während dieser anfänglichen Zyklen kann eine Grundbasislinie generiert werden. Die maximale Zyklenanzahl kann ein Abbruchkriterium für den Fall bilden, dass keine Amplifikation bei der Probe detektierbar ist. Diese Anzahl ist typischerweise die Anzahl der Zyklen, welche auch in einem klassischen PCR-Prozess vorgegeben wird.

**Fig. 2** illustriert die Auswertung (Schritt 15 in Fig. 1) der detektierten Fluoreszenzsignale. Diese Fluoreszenzsignale können in Bezug zu einzelnen PCR-Zyklen erfasst werden oder auch in Bezug zu bestimmten Zeitpunkten während des Amplifikationsprozesses, insbesondere in den Fällen von isothermen Amplifikationsprozessen (z.B. bei Gesamtgenomamplifikationen). Teilfigur A zeigt den Hintergrund (*background -* BG) oder eine Basislinie, die von einzelnen Datenpunkten (offene Kreise) gebildet wird, die insbesondere in frühen PCR-Zyklen gemessen werden und bei denen nicht von einer Amplifikation auszugehen ist. Der Rahmen um die einzelnen Datenpunkte repräsentiert einen geschätzten Hintergrund mit gewissen Toleranzen. Dieser so definierte Hintergrund ist die Basis für die Tests der nachfolgenden Datenpunkte auf der Grundlage der gemessenen Fluoreszenzsignale in folgenden PCR-Zyklen bzw. im folgenden Amplifikationsprozess. Teilfigur B stellt die nachfolgend gemessenen Datenpunkte als geschlossene Kreise dar, die auf weiteren Fluoreszenzsignalen in nachfolgenden PCR-Zyklen bzw. im nachfolgenden Prozess beruhen und die sich innerhalb des Rahmens des Hintergrundes befinden. Der jüngste Datenpunkt, der mit einem Kreuz dargestellt ist, stellt den aktuellen Messwert dar, der sich ebenfalls innerhalb des Rahmens des Hintergrundes befindet. Hier ist davon auszugehen, dass keine Amplifikation stattgefunden hat. Es wird also zunächst auf der Basis der Datenpunkte aus vorangegangenen Zyklen der alte Hintergrund berechnet, das heißt, es wird der Hintergrund für alle Punkte mit Ausnahme des aktuellen Messwerts ermittelt (BG1). Wenn der aktuelle Datenpunkt vorliegt, wird ein zweiter Hintergrund BG2 berechnet, wobei der aktuelle Datenpunkt einbezogen wird. Nun kann statistisch getestet werden, ob sich die beiden möglichen Hintergründe BG1 und BG2 signifikant unterscheiden. Die statistische Auswertung kann gemäß folgender Vorgabe erfolgen:
Hypothese H1: BG1 = BG2
Hypothese H0: BG1 ≠ BG2
Ist wie in Teilfigur B P(H1) > P(H0), liegt kein signifikanter Unterschied vor und es hat keine Amplifikation stattgefunden. Der Amplifikationsprozess wird weitergeführt. Verändert sich hingegen durch den aktuellen Datenpunkt der Hintergrund signifikant (P(H1) < P(H0)), ist von einer Amplifikation auszugehen, wie in Teilfigur C dargestellt ist. Diese Information stellt die Grundlage für das weitere Vorgehen bei dem Amplifikationsprozess dar und der Prozess kann beendet werden.

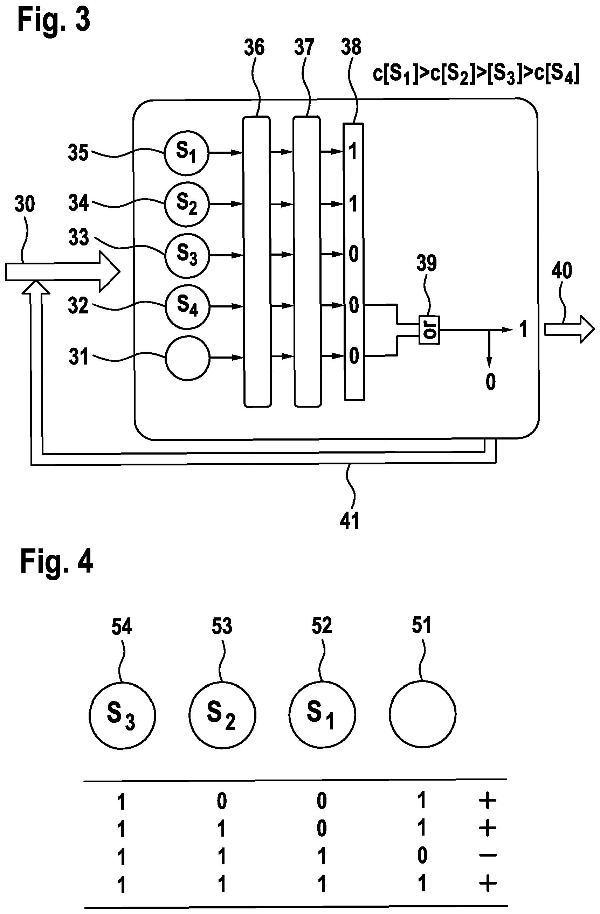

**Fig. 3** illustriert die Ausführung des Amplifikationsprozesses, mit dem eine Anfangskonzentration einer Proben-DNA bestimmt wird. Dieses Beispiel wird anhand eines PCR-Prozesses erläutert. Dieses und die nachfolgenden Beispiele können auch beispielsweise auf isotherme Amplifikationsprozesse angewendet werden, wobei die beobachteten Amplifikationssignale dann nicht einzelnen PCR-Zyklen sondern diskreten Zeitpunkten im Amplifikationsprozess zugeordnet werden. Parallel zur Probe 31 werden verschiedene Reaktionsansätze mit Standards 32, 33, 34 und 35 als Vergleichsproben mitgeführt. Hierbei repräsentiert der Standard 35 die größte Standardkonzentration S₁ und der Standard 32 die kleinste Standardkonzentration S₄. Zwischen der maximalen und der minimalen Standardkonzentration können im Prinzip beliebig viele Zwischenstufen der Standardkonzentrationen gewählt werden. In diesem Beispiel sind es zwei Konzentrationen S₂ und S₃. Die Anzahl der verschiedenen Standardkonzentrationen S₁ bis Sₙ bestimmt die Auflösung der Konzentrationsbestimmung. Alle Ansätze werden nach dem Start 30 des PCR-Prozesses parallel gefahren und während der einzelnen PCR-Zyklen werden die Signale der Amplifikation im Schritt 36 erfasst. Im Schritt 37 wird ausgewertet, ob auf eine Amplifikation rückzuschließen ist oder nicht. Dies kann insbesondere anhand des Verfahrens erfolgen, wie es im Zusammenhang mit Fig. 2 beschrieben wurde. Die im Feld 38 dargestellten Ziffern 1 und 0 stehen für eine Einstufung als Amplifikation ("1") oder keine Amplifikation ("0"). Der PCR-Prozess kann abgebrochen werden, wenn bei der Probe 31 eine Amplifikation festgestellt wird. Im Vergleich mit den Amplifikationsergebnissen bei den Standardproben 32 bis 35 kann dann rückgeschlossen werden, in welchem Konzentrationsintervall sich die Anfangskonzentration der DNA in der Probe 31 befand. Falls bei der Probe 31 keine Amplifikation festzustellen war aber bereits bei der geringsten Standardkonzentration 35 eine Amplifikation auftrat, kann der PCR-Prozess ebenfalls abgebrochen werden, da die Ausgangskonzentration in der Probe 31 unterhalb der Nachweisgrenze liegt, die durch die minimale Standardkonzentration 35 definiert ist. Diese Vorgehensweise wird durch die Abfrage 39 realisiert, indem zwischen der Probe 31 und der Vergleichsprobe 32 beziehungsweise dem Standard mit der geringsten Konzentration geprüft wird, ob bei einer der beiden Ansätze eine Amplifikation festgestellt wurde. In diesem Fall wird der PCR-Prozess beendet (Schritt 40). Ist weder bei der Probe 31 noch bei dem Standard S4 mit der geringsten Konzentration 32 eine Amplifikation festzustellen, wird der nächste PCR-Zyklus im Schritt 41 ausgeführt. Dieses Verfahren erlaubt eine eindeutige Zuweisung eines Konzentrationsintervalls. Die Konzentrationsintervalle sind dabei definiert durch die Anzahl der Standards. Zu erwarten ist dabei, dass die Standards S₁ bis Sₙ von der größten bis zur geringsten Konzentration aufeinanderfolgend mit fortschreitendem PCR-Prozess Amplifikationssignale liefern. Wird eine Amplifikation bei der Probe 31 festgestellt und gleichzeitig eine Amplifikation bei den Standards S₁ bis Sᵢ (i < n), so liegt die Ausgangskonzentration bei der Probe 31 im Intervall [Sᵢ, Sᵢ₊₁]. Stimmt das Feststellen einer Amplifikation bei den Standards nicht mit deren Konzentrationsreihenfolge überein, liegt kein valider Test vor. Wenn also ein Ansatz mit einer geringeren Standardkonzentration eine Amplifikation bei einem PCR-Zyklus zeigt, bei dem ein Standard mit einer höheren Konzentration noch keine Amplifikation zeigt, sind die Reaktionen nicht gleich effizient beziehungsweise nicht vergleichbar. Die Wahl der Standardkonzentrationen kann beispielsweise so vorgenommen werden, dass sie jeweils um einen Faktor 10 voneinander abweichen. Dies entspricht einer Quantifizierung im Sinne einer klassischen Echtzeit-PCR.

**Fig. 4** illustriert das Verfahren anhand einer Indikatorenvektordarstellung für einen Infektionsnachweis. Neben der eigentlichen Probe 51 werden drei Standards 52, 53, 54 mitgeführt, wobei der Standard 52 der Standard S₁ mit der geringsten Konzentration der nachzuweisenden DNA, der Standard 53 der Standard S₂ mit einer mittleren Konzentration der nachzuweisenden DNA und der Standard 54 der Standard S₃ mit einer maximalen Menge der nachzuweisenden DNA ist. Der Standard S₁ repräsentiert die Nachweisgrenze. Diese Nachweisgrenze stellt das späteste Abbruchkriterium der Reaktion dar. Wird bei der Probe 51 früher eine Amplifikation festgestellt und entspricht die Reihenfolge des Auftretens der Amplifikation bei den Standards deren Konzentrationsreihenfolge, wird der Test als positiv gewertet. Fig. 4 fasst die Auswertungen, ob die Reaktion bei einem bestimmten PCR-Zyklus als Amplifikation zu werten ist oder nicht, in einem Indikatorenvektor *I* zusammen. Jedes Reaktionsgefäß beziehungsweise jeder PCR-Ansatz (Proben und Standards) hat in diesem Vektor einen Eintrag, der nach jedem Zyklus neu evaluiert wird. Eine Reaktion wird als "Amplifikation" bewertet, wenn ein Signal über dem Hintergrund detektierbar ist. Dieser wird im Indikatorenvektor *I* der Indikatorenwert 1 (*true*) zugeordnet. Falls keine Amplifikation feststellbar ist, wird der Indikator der Reaktion auf 0 (false) gesetzt. In diesem Beispiel ist der Standard S₃ der größte Standard und wird als obere Nachweisgrenze links aufgeführt. Der zweite Standard S₂, der mengenmäßig zwischen dem größten und dem kleinsten Standard liegt, folgt als Nächstes. An dritter Position folgt der kleineste Standard S₁, welcher die Nachweisgrenze darstellt. Als letzter Eintrag im Zustandsvektor folgt die eigentliche Probe 51. Initialisiert wird das Experiment mit *I* = [0,0,0,0]. In der Fig. 4 sind die vier Vektoren gezeigt, welche einen validen Test darstellen. Alle zwölf anderen möglichen Fälle sind nicht zulässig und der Test müsste als nicht valide rapportiert werden. Beim Fall *I* = [1,1,1,0], liegt das Signal im Bereich der Nachweisgrenze. In diesem Fall können wegen Rauschen der Reaktionseffizienz gegebenenfalls ein oder mehr Zyklen angehängt werden, damit möglicherweise vorhandene kleine Unterschiede zwischen den einzelnen Reaktionsgefäßen zu keinem Fehler im Testentscheid führen. In der letzten Spalte der Darstellung ist für die jeweiligen Vektoren das Testergebnis als positiv (+) oder als negativ (-) dargestellt. Sobald einer dieser Vektoren vorliegt, kann die Reaktion abgebrochen werden.

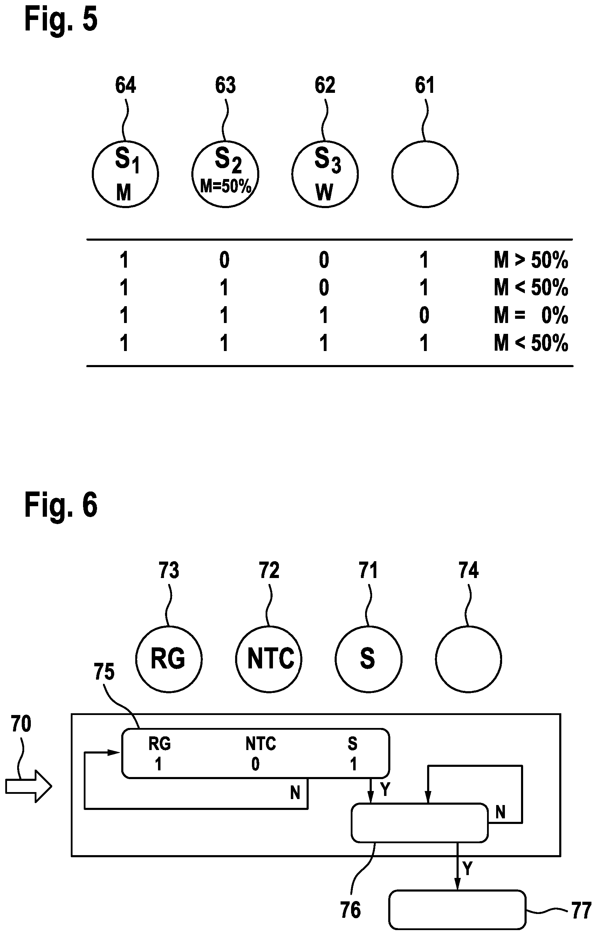

**Fig. 5** illustriert die Ausführung des Verfahrens für die Anwendung eines Mutationsnachweises, ebenfalls als Indikatorenvektordarstellung. Bei einem Mutationsnachweis wird in der Regel eine vordefinierte Menge der Proben-DNA in der zu untersuchenden Probe 61 eingesetzt. Da die Menge vordefiniert ist, wird in den Standards 62, 63 und 64 immer die gleiche Menge Standard-DNA beziehungsweise Referenz-DNA eingesetzt. Der Standard S₁ 64 enthält eine vorgelegte Template-DNA, bei welcher 100% die nachzuweisende Mutation aufweist (M). Dieser Standard S₁ bildet die Obergrenze, bei der als Erstes eine Amplifikation detektiert werden sollte. Die Untergrenze und damit das letzte Abbruchkriterium der Reaktion ist ein Standard S₃ 62, der 100% Wildtyp-Template (W) enthält. Zwischen diesen beiden Grenzen können nun mehrere Mischungsverhältnisse von Mutations- und Wildtyp-DNA gewählt werden. In diesem Beispiel ist ein weiterer Standard S₂ 63 vorgesehen, der 50% Mutation enthält (M = 50%). Die Einstellung von Mischungsverhältnissen von Mutations- und Wildtyp-DNA erlaubt das Einteilen der Probe in Anteilsbins, analog zu Histogrammen. Der hier gewählte Standard S₂ mit M = 50% erlaubt eine Kategorisierung des Mutationsanteils mit mehr als 50% und weniger als 50%. Bei diesem Ansatz kann so z. B. die Ploidität des Gens bestimmt werden. Feinere Unterteilungen werden durch das Einfügen von weiteren Standards sowie ein nummerisches Abschätzen der Effizienz erreicht. Wie im vorherigen Beispiel anhand der Fig. 4 erläutert, wird die Reaktion über den Zustandsvektor *I* kontrolliert und Testentscheide entsprechend gefällt.

**Fig. 6** illustriert das erfindungsgemäße Verfahren im Zusammenhang mit einer Gesamtgenomamplifikation. Bei einer Gesamtgenomamplifikation werden sämtliche in einer Probe vorkommenden Sequenzen amplifiziert, also nicht nur definierte, über Primer adressierte DNA-Sequenzen. In einer klassischen Gesamtgenomamplifikation werden keine Fluoreszenzsonden wie üblicherweise in einer Echtzeit-PCR eingesetzt werden, sondern das entstandene Amplifikationsprodukt wird durch den Einsatz von speziellen Farbstoffen visualisiert und quantifiziert. Diese speziellen Farbstoffe (z.B. PicoGreen^{®}, SYBR^{®} Green) lagern sich in doppelsträngige DNA ein und leuchten dabei stärker, so dass ein Anstieg in der Fluoreszenz auf eine erfolgte Amplifikation hindeutet. Wenn also ein Fluoreszenzsignal detektierbar ist, deutet dies auf das Vorhandensein von doppelsträngiger DNA hin, so dass dies also Amplifikation gewertet werden kann. Dieser Farbstoff wird den Reaktionsgemischen in den Ansätzen für den Prozess in einer definierten Menge zugemischt. Neben der eigentlichen Probe 71 (S) werden bei dem Prozess für die Gesamtgenomamplifikation drei weitere Vergleichsproben 72, 73 und 74 mitgeführt. Die Vergleichsprobe 72 enthält keine Template-DNA als sogenannte *no template control* (NTC). Bei dieser Probe sollte keine Amplifikation festzustellen sein, da keine zu amplifizierende DNA vorliegt. Als weitere Vergleichsprobe 73 wird die DNA eines Referenzgenoms (RG) mitgeführt. Dieses Referenzgenom ist zweckmäßigerweise Spezies-spezifisch. Wenn z. B. ein menschliches Genom insgesamt amplifiziert werden soll, wird als Referenzgenom das Genom eines oder mehrere anderer Menschen verwendet. Die eingesetzte Menge des Referenzgenoms entspricht beispielsweise der maximal einsetzbaren Menge für ein geeignetes Gesamtgenomamplifikationssystem. Dieses Referenzgenom in der Vergleichsprobe 73 sollte daher als erstes ein Amplifikationssignal liefern. Nach dem Start 70 wird die Reaktion so lange durchgeführt und so lange auf Amplifikation getestet, bis das Referenzgenom 73 und die Probe 71 im Zustandsvektor positiv sind (Zustand 75). Bei dem Zustand 75 zeigen also sowohl Referenzgenom 73 als auch Probe 71 eine Amplifikation und die NTC-Kontrolle 72 zeigt keine Reaktion bzw. Amplifikation. Nur bei diesem Zustand ist ausreichend DNA in der Probe vorhanden, und bis zu diesem Zustand hat keine unspezifische Primer-Amplifikation (Entstehung von Primer-Dimeren) stattgefunden, wie anhand der Kontrolle 72 gezeigt wurde. Dieser Zustand stellt einen ersten Checkpoint dar. Wurden die Bedingungen für den ersten Checkpoint erfüllt, wird die Reaktion fortgesetzt, nun aber auf ein neues Abbruchkriterium 76 getestet. Das neue Testkriterium 76 ist der Vergleich der Intensität der Amplifikation bei der Probe 71 und der Mengenreferenz 74. Diese Mengenreferenz 74 enthält die gewünschte Menge an Referenzgenom, wobei dies der gewünschten Menge an Produkt bei der Gesamtgenomamplifikation entspricht. Hierbei enthält die Mengenreferenz 74 alle Komponenten im WGA-Ansatz wie die anderen Ansätze mit Ausnahme des Amplifikationsenzyms. Dies hat zur Folge, dass in der Mengenreferenz 74 während der Reaktion keine Amplifikation, also keine DNA-Synthese stattfindet. Es wird lediglich ein Referenzfluoreszenzsignal durch den vorgelegten Fluoreszenzfarbstoff geliefert. Haben nun amplifizierte Probe 71 und die Mengenreferenz 74 die gleiche Fluoreszenzintensität, kann davon ausgegangen werden, dass im Prinzip die gleiche Menge an doppelsträngiger DNA in beiden Ansätzen vorliegt, so dass die Reaktion im Schritt 77 abgebrochen werden kann. Als weiteres Abbruchkriterium kann vorgesehen sein, dass die NTC-Kontrolle 72 ein Amplifikationssignal zeigt.

Dieses Verfahren kann auch auf eine spezifische, zielgerichtete Präamplifikation angewandt werden, bei der die bei einer Präamplifikation synthetisierte Menge an DNA kontrolliert wird. Dabei werden anstelle des Gesamtgenoms spezifische Primer eingesetzt, wodurch spezifische Genabschnitte entsprechend hochkopiert werden. Als Sonde kann hier anstelle eines Farbstoffs, der sich an doppelsträngige DNA einlagert, auch eine spezifische fluoreszenzmarkierte Sonde, die ein Fluoreszenzsignal in Abhängigkeit von synthetisierter DNA generiert, beispielsweise eine TaqMan^{®}-Sonde mit Fluorophor und Quencher, eingesetzt werden. Die Mengenreferenz beinhaltet dann die gewünschte Zielmenge an Amplifikat, eine äquivalente Menge an gespaltener Sonde, das heißt die gleiche Menge freier Fluorophore und Quencher, und eine komplementäre Restmenge der Sonde. Dem liegt zugrunde, dass bei einem Echtzeit-PCR-Ansatz in einem TaqMan^{®}-Sondensystem eine definierte Ausgangsmenge der Sonden (N₀ = c₀V) vorgegeben wird. Wenn die Amplifikation beginnt, wird die Sonde gespalten. Die Menge an Sonden und freien Fluorophoren ist dann abhängig von der entstandenen Kopienanzahl N_{Amplicon}. Die restlichen Sonden N_{S} können mit N_{S}=N₀-N_{Amplicon} berechnet werden. Anstelle einer NTC-Kontrolle wird als Abbruchkriterium eine weitere Referenz mitgeführt, die eine Nachweisgrenze für die Amplifikation (LoA - *limit of amplification*) detektierbar macht. Hierbei wird eine minimal einzusetzende Genomverdünnung verwendet. Der erste Checkpoint ist hierbei also der Amplifikationszeitpunkt, bei dem eine Assay-spezifische, vordefinierte Genomverdünnung, also die Referenz LoA, amplifiziert wurde.

Die Verfahren der Gesamtgenomamplifikation gemäß den Erläuterungen zu Fig. 6 und der Mutationsnachweis gemäß den Erläuterungen zu Fig. 5 können miteinander verknüpft werden und als kontrollierter Arbeitsablauf für einen Mutationsnachweis ausgestaltet werden. Hierbei können mikrofluidische Systeme und/oder Pipettierroboter eingesetzt werden. Ein derartiger vollautomatischer Arbeitsablauf kann insbesondere im Zusammenhang mit mikrofluidischen Systemen eine sehr vorteilhafte Einsatzmöglichkeit des erfindungsgemäßen Verfahrens bieten, welche beispielsweise in *Point-of-Care*-Anwendungen eingesetzt werden kann.

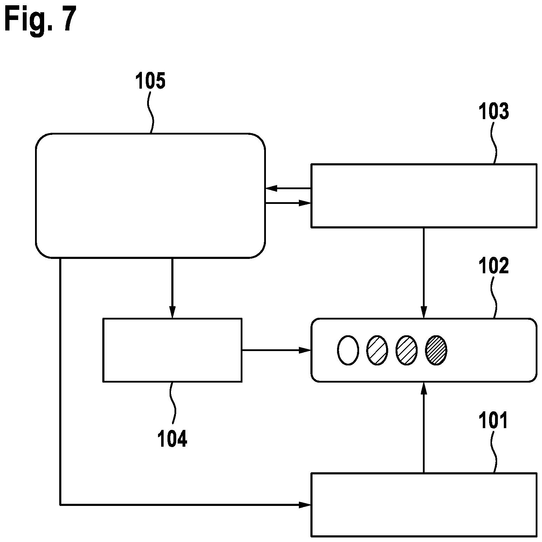

**Fig. 7** illustriert die Gerätekomponenten, die für die beschriebenen Echtzeit-PCR-Prozesse eingesetzt werden können. Die Grundlage bildet hierbei ein Gerät, das eine optofluidische Echtzeit-PCR ermöglicht und also eine Signalauslesung optischer Signale erlaubt, um anhand von Fluoreszenzsignalen die Amplifikation in den einzelnen Proben beziehungsweise PCR-Reaktionen beobachten zu können. Ein solches Gerät umfasst ein Heiz- und Kühlsystem 101 (Thermocycler), das mit den verschiedenen PCR-Reaktionsansätzen 102 wechselwirkt. Darüber hinaus verfügt das Gerät über eine optische Einheit 103, mit der die Auslesung der Amplifikationssignale erfolgt. Darüber hinaus kann eine Einrichtung für ein Fluidhandling 104 vorgesehen sein, beispielsweise ein Robotersystem oder ein entsprechendes mikrofluidisches System. Insgesamt ist es vorteilhaft, ein solches System als mikrofluidisches System auszugestalten, da ein mikrofluidisches System mit sehr kleinen Probenvolumina betrieben werden kann und eine Teil- oder Vollautomatisierung erlaubt. Das System ist darüber hinaus mit einer Reaktionskontrolleinheit 105 ausgestattet, mit der eine in-situ-Auswertung der optischen Daten erfolgt. Zur Realisierung des rückgekoppelten Echtzeit-PCR-Systems der vorliegenden Erfindung ist die Reaktionskontrolleinheit 105 so ausgestaltet, dass sie mit allen Einheiten des Systems wechselwirken kann. Die Reaktionskontrolleinheit 105 kann insbesondere steuern, dass in Abhängigkeit der beobachteten Signale der Amplifikation die Anzahl der PCR-Zyklen dynamisch eingestellt wird.

## Patentansprüche

1. Verfahren zur Durchführung eines Prozesses zur Amplifikation von Nukleinsäuren (10), wobei Proben-Nukleinsäuren (31; 51; 61; 71) und Referenz-Nukleinsäuren (32-35; 52-54; 62-64, 72-73) in getrennten Reaktionsansätzen amplifiziert werden, wobei Signale der Amplifikation in Echtzeit beobachtet werden und die Anzahl von Amplifikationszyklen und/oder die Dauer des Amplifikationsprozesses in Abhängigkeit von Signalen der Amplifikation dynamisch eingestellt werden, **dadurch gekennzeichnet, dass** das Verfahren für eine Gesamtgenomamplifikation eingesetzt wird, wobei wenigstens eine erste Vergleichsprobe (73) mit definierter Konzentration an Nukleinsäure eines Referenzgenoms mitgeführt wird und die eingesetzte Menge des Referenzgenoms der maximal einsetzbaren Menge von DNA für Gesamtgenomamplifikationssysteme entspricht.

2. Verfahren nach Anspruch 1, **dadurch gekennzeichnet, dass** die Amplifikation von Nukleinsäuren im Rahmen einer Echtzeit-PCR durchgeführt wird und dass die Zyklen zur Amplifikation PCR-Zyklen sind.

3. Verfahren nach Anspruch 1 oder Anspruch 2, **dadurch gekennzeichnet, dass** die Beobachtung und/oder Auswertung der Signale der Amplifikation in Echtzeit startet, wenn eine vorgebbare Mindestanzahl von Amplifikationszyklen und/oder eine vorgebbare Mindestdauer des Amplifikationsprozesses durchgeführt worden ist.

4. Verfahren nach einem der vorhergehenden Ansprüche, **dadurch gekennzeichnet, dass** der Prozess beendet wird, wenn die Signalstärke der Amplifikation der Proben-Nukleinsäuren die Signalstärke der Amplifikation der Referenz-Nukleinsäuren erreicht und/oder übersteigt.

5. Verfahren nach einem der vorhergehenden Ansprüche, **dadurch gekennzeichnet, dass** der Prozess abgebrochen wird, wenn eine vorgebbare Maximalanzahl von Amplifikationszyklen und/oder eine vorgebbare Maximaldauer des Amplifikationsprozesses durchgeführt worden ist.

6. Verfahren nach einem der vorhergehenden Ansprüche, **dadurch gekennzeichnet, dass** die Signale der Amplifikation in Bezug zu jeweils durchgeführten Amplifikationszyklen und/oder in Bezug zu festlegbaren Zeitpunkten beobachtet werden und bei einem signifikanten Anstieg der Signale der jeweilige Zyklus oder der jeweilige Zeitpunkt als "Amplifikation" eingestuft und ein Vergleich der Einstufung zwischen Proben-Nukleinsäuren und Referenz-Nukleinsäuren für eine Auswertung herangezogen wird.

7. Verfahren nach Anspruch 6, **dadurch gekennzeichnet, dass** die Ergebnisse des Prozesses zur Amplifikation als Indikatorenvektordarstellung ausgewertet werden, wobei die als "Amplifikation" eingestuften Zyklen oder Zeitpunkte dem Indikatorenwert "1" und die anderen Zyklen oder Zeitpunkte dem Indikatorenwert "0" zugeordnet werden.

8. Verfahren nach einem der vorhergehenden Ansprüche, **dadurch gekennzeichnet, dass** mit dem Verfahren die Ausgangsmenge der Proben-Nukleinsäuren (31) ermittelt und/oder kontrolliert wird, wobei wenigstens zwei Vergleichsproben (32 - 35) mit definierter Ausgangsmenge der Referenz-Nukleinsäuren parallel mitgeführt werden

9. Verfahren nach einem der vorhergehenden Ansprüche, **dadurch gekennzeichnet, dass** das Verfahren als Infektionsnachweis eingesetzt wird, wobei wenigstens eine Vergleichsprobe (52) mit einer Konzentration der nachzuweisenden Nukleinsäure parallel mitgeführt wird, die eine untere Nachweisgrenze für den Infektionsnachweis repräsentiert.

10. Verfahren nach einem der vorgehenden Ansprüche, **dadurch gekennzeichnet, dass** das Verfahren als Mutationsnachweis eingesetzt wird, wobei eine Vergleichsprobe (64) mit definierter Konzentration an Nukleinsäure, die einen Anteil der nachzuweisenden Mutation von 100%, und eine Vergleichsprobe (62) mit definierter Konzentration an Nukleinsäure, die einen Anteil der nachzuweisenden Mutation von 0% enthält, mitgeführt werden.

11. Verfahren nach einem der vorgehenden Ansprüche, **dadurch gekennzeichnet, dass** zusätzlich eine zweite Vergleichsprobe (72) ohne zu amplifizierender Nukleinsäure und/oder eine dritte Vergleichsprobe (74) mit einer definierten Menge an Nukleinsäure des Referenzgenoms mitgeführt werden, wobei die definierte Menge der dritten Vergleichsprobe der gewünschten Zielmenge an Amplifikationsprodukt bei der Gesamtgenomamplifikation entspricht, und wobei der Reaktionsansatz der dritten Vergleichsprobe kein Amplifikationsenzym enthält, und wobei das Signal der Amplifikation auf der Verwendung von Fluoreszenzfarbstoffen beruht, die sich in doppelsträngige DNA einlagern.

## Claims

1. Method for carrying out a process for amplification of nucleic acids (10), wherein sample nucleic acids (31; 51; 61; 71) and reference nucleic acids (32-35; 52-54; 62-64, 72-73) are amplified in separate reactions, wherein signals from the amplification are observed in real time and the number of amplification cycles and/or the duration of the amplification process are dynamically adjusted according to signals from the amplification, **characterized in that** the method is used for total genome amplification, wherein at least a first comparative sample (73) having a defined concentration of nucleic acid of a reference genome is run at the same time and the amount of the reference genome used corresponds to the maximum usable amount of DNA for total genome amplification systems.

2. Method according to Claim 1, **characterized in that** the amplification of nucleic acids is carried out as a real-time PCR and **in that** the amplification cycles are PCR cycles.

3. Method according to Claim 1 or Claim 2, **characterized in that** the observation and/or evaluation of the signals from the amplification in real time starts when a specifiable minimum number of amplification cycles and/or a specifiable minimum duration of the amplification process has been carried out.

4. Method according to any of the preceding claims, **characterized in that** the process is ended when the signal strength of the amplification of the sample nucleic acids reaches and/or exceeds the signal strength of the amplification of the reference nucleic acids.

5. Method according to any of the preceding claims, **characterized in that** the process is terminated when a specifiable maximum number of amplification cycles and/or a specifiable maximum duration of the amplification process has been carried out.

6. Method according to any of the preceding claims, **characterized in that** the signals from the amplification are observed in relation to amplification cycles respectively carried out and/or in relation to definable time points and, when the signals rise significantly, the respective cycle or the respective time point is classified as "amplification" and a comparison of the classification between sample nucleic acids and reference nucleic acids is used for evaluation.

7. Method according to Claim 6, **characterized in that** the results of the process for amplification are evaluated as an indicator vector representation, wherein the cycles or time points classified as "amplification" are assigned to the indicator value "1" and the other cycles or time points are assigned to the indicator value "0".

8. Method according to any of the preceding claims, **characterized in that** the starting amount of the sample nucleic acids (31) is determined and/or checked by means of the method, wherein at least two comparative samples (32 - 35) having a defined starting amount of the reference nucleic acids are run in parallel at the same time.

9. Method according to any of the preceding claims, **characterized in that** the method is used as infection detection, wherein at least one comparative sample (52) having a concentration of the nucleic acid to be detected that represents a lower detection limit for the infection detection is run in parallel at the same time.

10. Method according to any of the preceding claims, **characterized in that** the method is used as mutation detection, wherein a comparative sample (64) having a defined concentration of nucleic acid containing a proportion of the mutation to be detected of 100% and a comparative sample (62) having a defined concentration of nucleic acid containing a proportion of the mutation to be detected of 0% are run at the same time.

11. Method according to any of the preceding claims, **characterized in that** a second comparative sample (72) without nucleic acid to be amplified and/or a third comparative sample (74) having a defined amount of nucleic acid of the reference genome are additionally run at the same time, wherein the defined amount of the third comparative sample corresponds to the desired target amount of amplification product in the total genome amplification, and wherein the reaction containing the third comparative sample does not contain an amplification enzyme, and wherein the signal from the amplification is based on the use of fluorescent dyes which are intercalated into double-stranded DNA.

## Revendications

1. Procédé de mise en œuvre d'un processus d'amplification d'acides nucléiques (10), des acides nucléiques d'échantillon (31 ; 51 ; 61 ; 71) et des acides nucléiques de référence (32-35 ; 52-54 ; 62-64, 72-73) étant amplifiés dans des mélanges réactionnels séparés, des signaux de l'amplification étant observés en temps réel et le nombre de cycles d'amplification et/ou la durée du processus d'amplification étant réglés de manière dynamique en fonction de signaux de l'amplification, **caractérisé en ce que** le procédé est utilisé pour une amplification de génome total, au moins un premier échantillon comparatif (73) présentant une concentration définie en acide nucléique d'un génome de référence étant entraîné et la quantité utilisée du génome de référence correspondant à la quantité maximale utilisable d'ADN pour les systèmes d'amplification de génome total.

2. Procédé selon la revendication 1, **caractérisé en ce que** l'amplification d'acides nucléiques est mise en œuvre dans le cadre d'une PCR en temps réel et **en ce que** les cycles d'amplification sont des cycles de PCR.

3. Procédé selon la revendication 1 ou 2, **caractérisé en ce que** l'observation et/ou l'évaluation des signaux de l'amplification en temps réel démarre lorsqu'un nombre minimal pouvant être prédéfini de cycles d'amplification et/ou une durée minimale pouvant être prédéfinie du processus d'amplification a/ont été mis(e) en œuvre.

4. Procédé selon l'une des revendications précédentes, **caractérisé en ce que** le processus est arrêté lorsque l'intensité de signal de l'amplification des acides nucléiques d'échantillon atteint et/ou dépasse l'intensité de signal de l'amplification des acides nucléiques de référence.

5. Procédé selon l'une des revendications précédentes, **caractérisé en ce que** le processus est interrompu lorsqu'un nombre maximal pouvant être prédéfini de cycles d'amplification et/ou une durée maximale pouvant être prédéfinie du processus d'amplification a/ont été mis(e) en œuvre.

6. Procédé selon l'une des revendications précédentes, **caractérisé en ce que** les signaux de l'amplification sont observés par rapport aux cycles d'amplification à chaque fois mis en œuvre et/ou par rapport à des moments pouvant être fixés et, lors d'une augmentation significative des signaux, le cycle respectif ou le moment respectif est classé comme "amplification" et une comparaison du classement entre les acides nucléiques d'échantillon et les acides nucléiques de référence étant utilisée pour une évaluation.

7. Procédé selon la revendication 6, **caractérisé en ce que** les résultats du processus d'amplification sont évalués comme représentation vectorielle d'indicateurs, les cycles ou les moments classés comme "amplification" recevant la valeur d'indicateur "1" et les autres cycles ou moments recevant la valeur d'indicateur "0".

8. Procédé selon l'une des revendications précédentes, **caractérisé en ce que** le procédé permet de déterminer et/ou de contrôler la quantité de départ des acides nucléiques d'échantillon (31), au moins deux échantillons comparatifs (32-35) présentant une quantité de départ définie des acides nucléiques de référence étant entraînés en parallèle.

9. Procédé selon l'une des revendications précédentes, **caractérisé en ce que** le procédé est utilisé pour une détection d'une infection, au moins un échantillon comparatif (52) présentant une concentration en acide nucléique à détecter, qui représente une limite de détection inférieure pour la détection d'une infection, étant entraîné en parallèle.

10. Procédé selon l'une des revendications précédentes, **caractérisé en ce que** le procédé est utilisé pour une détection d'une mutation, un échantillon comparatif (64) présentant une concentration définie en acide nucléique qui contient une proportion de la mutation à détecter de 100% et un échantillon comparatif (62) présentant une concentration définie en acide nucléique qui contient une proportion de la mutation à détecter de 0% étant entraînés.

11. Procédé selon l'une des revendications précédentes, **caractérisé en ce qu'**en plus, un deuxième échantillon comparatif (72) sans acide nucléique à amplifier et/ou un troisième échantillon comparatif (74) présentant une quantité définie d'acide nucléique du génome de référence est/sont entraîné(s), la quantité définie du troisième échantillon comparatif correspondant à la quantité cible souhaitée de produit d'amplification lors de l'amplification de génome total et le mélange réactionnel du troisième échantillon comparatif ne contenant pas d'enzyme d'amplification et le signal de l'amplification reposant sur l'utilisation de colorants fluorescents qui s'incorporent dans l'ADN double brin.
